**Europäisches Patentamt**

**European Patent Office**

**Office européen des brevets**

(11) Veröffentlichungsnummer: **0 025 911**
**A1**

(12) **EUROPÄISCHE PATENTANMELDUNG**

(21) Anmeldenummer: **80105247.3**

(22) Anmeldetag: **03.09.80**

(51) Int. Cl.³: **A 61 C 9/00**
**A 61 C 19/04, A 61 B 5/10**

(30) Priorität: **12.09.79 DE 2936847**

(43) Veröffentlichungstag der Anmeldung:
**01.04.81 Patentblatt 81/13**

(84) Benannte Vertragsstaaten:
**AT BE CH DE FR GB IT LI NL SE**

(71) Anmelder: **Heitlinger, Paul, Dr.**
**Chemnitzer Strasse 15**
**D-6054 Rodgau 3 (Nieder-Roden)(DE)**

(71) Anmelder: **Rödder, Fritz**
**Schulstrasse 1**
**D-6273 Waldems/Esch(DE)**

(72) Erfinder: **Heitlinger, Paul, Dr.**
**Chemnitzer Strasse 15**
**D-6054 Rodgau 3 (Nieder-Roden)(DE)**

(72) Erfinder: **Rödder, Fritz**
**Schulstrasse 1**
**D-6273 Waldems/Esch(DE)**

(74) Vertreter: **Weber, Dieter, Dr. et al,**
**Patentanwälte Dr. D. Weber und Dipl.-Phys. K. Seiffert**
**Gustav-Freytag-Strasse 25**
**D-6200 Wiesbaden 1(DE)**

(54) Verfahren zur Herstellung von Zahnersatz und Vorrichtung zur Durchführung des Verfahrens.

(57) Zur Herstellung von Zahnersatz wird der präparierte Zahnstumpf in einem Arbeitsmodell nachgebildet und das von Hand an das Arbeitsmodell entsprechend angepaßte Ersatzstück geformt. Um die Arbeit des Zahnarztes zu erleichtern und gleichzeitig eine größere Genauigkeit zu erreichen, ist vorgesehen, daß die Nachbildung des Zahnstumpfes elektrooptisch derart erfolgt, daß dem Zahnstumpf entsprechende Abbildungsinformationssignale erzeugt werden, die in einem elektronischen Rechner (18) zu elektrischen und/oder magnetischen Steuersignale umgeformt werden, mit deren Hilfe eine Fräsmaschine (24) das Arbeitsmodell aus einem Materialblock selbsttätig herausfräst, und/oder daß das positive Ersatzstück von der Fräsmaschine (24) nach Steuersignalen aus einem Materialblock herausgefräst wird, die aus Abbildungsinformationssignalen abgeleitet sind, welche elektrooptisch von dem handgeformten Ersatzmodell abgeleitet sind.

Die Vorrichtung zur Durchführung des Verfahrens ist gekennzeichnet durch eine optische Abbildungsaufnahmeeinrichtung (3) zum Bestreichen der betreffenen Zahnpartie, ferner einen elektronischen Rechner (18) zum Abtasten und Auswerten der von einer Abbildungseinrichtung erzeugten Abbildungsinformationssignale, eine Fräsmaschine (24) und/oder gegebenenfalls ein Speichergerät.

Fig. 3

0025911

Dr. Paul Heitlinger, Chemnitzer Straße 15
6054 Rodgau 3 (Nieder-Roden)
u n d
Fritz Rödder, Schulstraße 1, 6273 Waldems/Esch

- - - - - - - - - - - - - - - - - - - - - - - - -

Verfahren zur Herstellung von Zahnersatz und
Vorrichtung zur Durchführung des Verfahrens

- - - - - - - - - - - - - - - - - - - - - - - - -

Die Erfindung betrifft ein Verfahren zur Herstellung
von Zahnersatz, bei dem der präparierte Zahnstumpf in
einem Arbeitsmodell nachgebildet und das von Hand an
das Arbeitsmodell entsprechend angepaßte Ersatzstück
geformt wird, und sie bezieht sich auf eine Vorrichtung zur Durchführung des Verfahrens.

Es sind Verfahren der vorstehend genannten Art zur Herstellung von Zahnersatz vielfach bekannt. Die Fähigkeit,
eine exakt sitzende Krone (als Zahnersatz) herzustellen, hängt von der Beherrschung der Abdrucktechnik ab. Die drei gebräuchlichsten Verfahren sind die Abdrucknahme mit Silicon- oder Gummimasse, mit Hydrokolloid sowie mit einem Kupferring und Abdruckmasse. Die Arbeit des Zahnarztes und seine Ausbildung werden allein schon dadurch erschwert, daß er unbedingt mehrere Methoden zur Abdrucknahme beherrschen muß. Außerdem muß für die Abdrucknahme das elastische Material, z. B. das Zahnfleisch, ausreichend zurückgezogen werden, damit die oberen Ränder der später präparierten Zähne bzw. Zahnstümpfe mit erfaßt werden. Die Weichteilretraktion erfordert einen nicht unerheblichen Arbeitsaufwand.

Ferner ist es wichtig, in Fällen, in denen im Frontbereich mehrere Kronen benötigt werden, die Zähne zu präparieren und dann mit einem gut sitzenden temporären Ersatz zu versehen. Bei den genannten Maßnahmen sind meist mehrere Sitzungen und Abdrücke nötig.

Das neue Abdruckverfahren ist die Methode mit Kupferring und Abdruckmasse. Der Nachteil des benutzten Materials besteht darin, daß mit ihm nicht gleichzeitig ein Abdruck von der gesamten Zahnreihe genommen werden kann, kein Abdruck von unter sich gehenden Partien genommen werden kann und die ziemlich warme Abdruckmasse eine Reizung der Pulpa nach sich ziehen kann. Das Formen und das Einsetzen des Ringes muß ausserordentlich vorsichtig geschehen, damit der Weichteilansatz nicht geschädigt wird. Im Falle multipler Pfeilerzähne ist die Abdrucknahme sehr zeitraubend.

Die zweite Methode erfolgt mit dem Gummimassenmaterial. In den fünfziger Jahren wurden mit Polysulfid-, Silicon- und Polyäthergummi drei Arten von elastischem oder Abdruckmaterial auf Gummibasis entwickelt. Silicon- und Polyäthergummi haben zwar eine bessere Farbe und einen angenehmeren Geschmack und sind in ästhetischer Hinsicht akzeptabler, sie werden aber nicht so häufig benutzt wie Polysulfidgummi.

Der Zeitaufwand ist bei Verwendung gummielastischer Materialien groß. Ihre Verwendung hat aber hauptsächlich den Nachteil, daß die Weichteilretraktion und eine völlige Trockenheit des betreffenden Gebietes notwendig sind, da das Abdruckmaterial sehr leicht verschoben werden kann.

In einer Untersuchungsreihe von Ferguson und Strode an Gummimassenabdrücken wiesen 10 % von 250 Abdrücken Lufteinschlüsse unter der Oberfläche auf, die nach Wegfall des Druckes sich ausdehnend Einsenkungen in der Matrize und Vorsprünge an der Krone verursachen. Das vorzeitige Abnehmen des Abdruckes ist eine häufige Ursache des Mißerfolges. Die Zeit zwischen dem Beginn des Mischens und dem Abnehmen des Abdruckes soll mindestens 10, besser noch 15 Minuten betragen. Es können hier aber sehr leicht Fehler und falsche Messungen auftreten.

Das dritte bekannte Verfahren erfolgt mit reversiblem Hydrokolloid. Dieses Abdruckmaterial wird etwa seit 1925 benutzt. Es wird beim Erhitzen flüssig und kehrt nach dem Abkühlen wieder in seinen Gelzustand zurück. Hauptnachteil von Hydrokolloid ist die Notwendigkeit, den Abdruck sofort auszugießen. Da Hydrokolloid das Zahnfleisch nicht verlagert, sind für die Genauigkeit der Wiedergabe des marginalen Bereiches die Weichteilretraktion und die sorgfältige Trocknung unerläßlich. Es versteht sich, welche Schwierigkeiten und welcher Zeitaufwand hierbei notwendig sind.

Zur Sicherstellung der Genauigkeit des Abdruckes sind folgende Richtlinien zu befolgen: Das Löffelmaterial muß zunächst temperiert werden, damit es eine gewisse gallertige Konsistenz annimmt. Dadurch wird die Viskosität erhöht und der Temperaturschock für den Zahn verringert. Außerdem muß der Abdrucklöffel okklusal und lateral eine 3 mm dicke Materialschicht aufweisen, damit eine hinreichende Genauigkeit gewährleistet ist.

Abgesehen von dem unangenehmen Geruch des Gummimassematerials ist auch dieses Verfahren entsprechend schwierig, zeitaufwendig und kompliziert. Für einen Abdruck von der gesamten Zahnreihe werden mindestens 5 Minuten benötigt. Das Modell muß zur Vermeidung von Formveränderungen innerhalb von 15 bis 40 Minuten gegossen werden. Man erkennt die Schwierigkeiten der bekannten Verfahren zum Herstellen von Zahnersatz, die ohne Abdruckverfahren nicht denkbar sind.

Es ergeben sich aber noch weitere Schwierigkeiten im Zusammenhang mit den temporären Maßnahmen. Jeder Patient ist nämlich an seinem Aussehen interessiert, nachdem die Präparation für die Überkronung abgeschlossen ist. Die alte Regel, daß ein temporärer oder provisorischer Ersatz nicht so schön sein muß wie der bleibende Ersatz, ist schlichtweg falsch. Gut hergestellte und sorgfältig angepaßte Ersatzformen erfüllen einen funktionellen und einen ästhetischen Zweck.

Hauptaufgabe einer jeglichen provisorischen Ersatzform ist der optimale Schutz für den Zahn und das ihn umgebende Gewebe für die Zeit, bis der endgültige Ersatz fertiggestellt ist. Der temporäre Ersatz bietet dem Zahnarzt die Möglichkeit, sich genau zu veranschaulichen, wie der endgültige Ersatz hinsichtlich seiner Form und Größe sowie in gewissem Maß auch hinsichtlich der Farbe aussehen muß. Der temporäre Ersatz ermöglicht es außerdem, die vorläufige Anordnung vor der Herstellung des endgültigen Ersatzes abzuändern. Der provisorische Ersatz schützt auch die Pulpa vor chemischen, thermischen und mechanischen Einflüssen.

Eines der heute noch gebräuchlichen älteren Verfahren ist die Alginattechnik zur Herstellung eines temporären Acrylatersatzes.

Auch beim temporären Ersatz muß zunächst mindestens ein Abdruck für die Herstellung des Arbeitsmodelles der präparierten Zahnstümpfe erstellt werden, danach muß von Hand ein Ersatzmodell aus Wachs oder dergleichen erstellt und mit dessen Hilfe der temporäre Ersatz modelliert werden.

Aufgabe der Erfindung ist daher die Verbesserung des bekannten Verfahrens zur Herstellung von Zahnersatz mit den eingangs erläuterten Merkmalen und Maßnahmen sowie die Schaffung einer Vorrichtung zur Durchführung eines solchen Verfahrens, mit dem die Arbeit des Zahnarztes erleichtert wird und gleichzeitig eine größere Genauigkeit erreicht wird.

Diese Aufgabe wird erfindungsgemäß dadurch gelöst, daß die Nachbildung des Zahnstumpfes elektrooptisch derart erfolgt, daß dem Zahnstumpf entsprechende Abbildungsinformationssignale erzeugt werden, die in einem elektronischen Rechner zu elektrischen und/oder magnetischen Steuersignalen umgeformt werden, mit deren Hilfe eine Fräsmaschine das Arbeitsmodell aus einem Materialblock selbsttätig herausfräst, und/oder daß das positive Ersatzstück von der Fräsmaschine nach Steuersignalen aus einem Materialblock herausgefräst wird, die aus Abbildungsinformationssignalen abgeleitet sind, welche elektrooptisch von dem handgeformten Ersatzmodell abgeleitet sind. Durch die neue Erfindung ist es möglich, den oder die Zahnstümpfe mit den benachbarten Kiefernbereichen elektrooptisch abzubilden, d.h. zu photographieren, zu filmen oder auf einer Röntgenplatte abzubilden. Aus einer solchen Nachbildung, die dem Zahnstumpf mit den benachbarten Bereichen entspricht, können Abbildungsinformationssignale erzeugt werden, indem z. B. optisch eine Photographie oder eine Röntgenaufnahme abgetastet wird. Der Abstand von Zeile zu Zeile erlaubt ersichtlich eine äußerst große Genauigkeit. Es bereitet durch die Anwendung der erfindungsgemäßen Maßnahmen keine Schwierigkeiten mehr, Nachbildungen des Zahnstumpfes auf $1\mu$ genau zu erstellen. Wenn durch den entsprechenden Abtaster die Abbildungsinformationssignale entsprechend erzeugt worden sind, gibt man diese einem elektronischen Rechner, indem sie zu elektrischen und/oder magnetischen Steuersignalen umgeformt werden. Es braucht hier auf die Konstruktion des elektronischen Rechners nicht im einzelnen eingegangen zu werden; denn es ist beim heutigen Stand der Technik möglich, entsprechende Informationssignale zu kodieren, zu verarbeiten und wieder zu dekodieren. Auf diese Weise kann man elektromagnetische, elektrische oder magnetische Steuersignale erhalten, die in ein entsprechendes Steueraggregat in einer Fräsmaschine eingegeben werden, welche, gesteuert von diesen Steuersignalen, das gewünschte Arbeitsmodell aus einem Materialblock selbsttätig herausfräst. Hierbei kann ein abriebfester Kunststoff, es kann aber auch Gips, ein Harz oder ein anderes gewünschtes Material verwendet werden, sofern das Abfräsen gewährleistet ist. In überraschender Weise kann man somit ohne die umständlichen und teilweise ungenauen Abdruckverfahren ein sehr genaues Arbeitsmodell erstellen.

Gleichzeitig, alternativ oder zusätzlich kann durch die erfindungsgemäße Lehre auch ein positives Ersatzstück ohne Adruckverfahren hergestellt werden. Die Fräsmaschine fräst das positive Ersatzstück, d. h. entweder der temporäre Zahnersatz oder eine Brücke oder dergleichen, aus einem entsprechenden Materialblock nach Steuersignalen, die ähnlich erhalten werden, wie vorstehend beschrieben. Man leitet sie nämlich aus Abbildungsinformationssignalen ab, die wiederum elektrooptisch, z. B. durch Photographieren, Filmen, Röntgenaufnahmen oder dergleichen von dem handgeformten Ersatzmodell abgeleitet sind. Dabei ist es besonders zweckmäßig, wenn erfindungsgemäß die elektrooptische Nachbildung des Zahnstumpfes und/oder Ersatzmodells photogrammetrisch oder durch Röntgenaufnahmen erfolgt und wenn die Abbildungsinformationssignale von einem Papierphoto, einem optischen Film, einer Röntgenplatte oder einem Videoband abgeleitet werden.

Es sind die verschiedensten Röntgengeräte zur Abbildung von Zähnen und Kiefern bekannt. Wenn z. B. ein Panoramaröntgengerät so verbessert und ausgestaltet wird, daß die Zähne nicht nur von einer Seite gesehen auf der Röntgenplatte erscheinen, sondern auch von unten, von der anderen Seite und überlappend auf mehreren Röntgenplatten aufgenommen sind, dann läßt sich das Verfahren der bekannten Photogrammetrie anwenden, so daß - in beliebige feine Raster bzw. Koordinaten unterteilt - die Röntgenaufnahmen in so feine Abbildungsinformationssignale umgewandelt werden können, daß damit praktisch eine sehr genaue räumliche Beschreibung des betreffenden Zahns bzw. Kiefers vorhanden ist.

Ingleicher Weise kann man aber den Zahnstumpf bzw. das präparierte Gebiß durch photographische Aufnahmen oder durch Filmen räumlich wiedergeben. Hierzu wird die bekannte Methode der Photogrammetrie eingesetzt. Es handelt sich hierbei bekanntlich um eine Verfahrenstechnik, nach der photographische Meßbilder hergestellt, gegebenenfalls geometrisch oder strukturell umgebildet und graphisch oder numerisch ausgewertet werden können. Die grundlegenden Verfahren für die Photogrammetrie wurden fast ausschließlich im Zusammenhang mit der Behandlung geodätisch-meßtechnischer Probleme entwickelt.

Für die Topographie hat die Photogrammetrie eine erhebliche Bedeutung, wobei hauptsächlich Luftbildmessungen eingesetzt werden. Man achtet dabei in bekannter Weise auf eine Längs- und Querüberdeckung zahlreicher, von einem Flugkörper hintereinander angefertigter Aufnahmen.

Benutzt man dieses ansich bekannte Verfahren bei der Zahntechnik, so kann man in überraschender Weise das präparierte Gebiß äußerst genau, sehr schnell und ohne die lästigen Behandlungen des Patienten nachbilden.

Zweckmäßig ist es erfindungsgemäß dabei, wenn die elektrischen und/oder magnetischen Steuersignale zwischengespeichert werden. Die ausgewerteten Abbildungsinformationssignale, die dann entweder direkt zur Steuerung der Fräsmaschine oder indirekt nach dem Zwischenspeichern zum Wiederauslesen verwendet werden können, ermöglichen auf engstem Raum das Anlegen von räumlichen Bildern mit größter Genauigkeit. Der Zahnarzt kann sozusagen im Vorübergehen das Gebiß seines Patienten räumlich und sehr genau festhalten, gibt die aufbereiteten Signale auf einen Speicher und kann diese dann jederzeit abrufen. Man erkennt auch die wirtschaftliche Bedeutung für die zahntechnischen Laboratorien. Der Materialaufwand wird durch das Entfallen der vielen Abdruckmassen geringer, es werden weniger Arbeitskräfte zum Modellieren gebraucht, und dennoch erreicht der Zahnarzt eine wesentlich bessere Genauigkeit, so daß ein von ihm erstellter Zahnersatz, z. B. eine über mehrere Zähne reichende Brücke, nach Fertigstellung beim Einpassen in das präparierte Gebiß des Patienten sofort genau paßt und einzementiert werden kann.

Vorteilhaft ist es gemäß der Erfindung ferner, wenn die Abbildungsinformationssignale für eine optisch sichtbare Abbildung auf einem Videoanzeigegerät ausgewertet werden. Durch die ansich bekannten Techniken auf dem Gebiet der Elektronik ist es möglich, die durch die vorstehend erwähnten Maßnahmen erhaltenen Abbildungsinformationssignale in elektrische und/oder magnetische Daten zu verarbeiten, die ohne weiteres auf einem Videoanzeigegerät optisch sichtbar gemacht werden. Diese Maßnahmen erleichtern das Präparieren des Gebisses des Patienten für den Zahnarzt, weil er ohne Materialaufwand für Abdruckmassen und ohne langwierige und für den Patienten unangenehme Eingriffe beim Handschleifen zwischendurch immer wieder den Ist-Zustand aus allen Perspektiven feststellen und überprüfen kann, so daß er von der Betrachtung mit den Spiegeln in der bislang üblichen Weise als alleiniges Hilfsmittel abgehen kann. Dieses Verfahren ist insbesondere nützlich, wenn mehrere Zähne nebeneinander zu präparieren sind. Er kann dann auf dem Videoanzeigegerät verschiedene Vergrößerungen einstellen und z. B. die Parallelstellung überprüfen, was er bislang nur nach Augenmaß tun konnte. Das bekannte Verfahren war ungenau und zeitraubend und wird in einfacher Weise durch das neue Verfahren genauer und schneller.

Weiterhin ist die Erfindung vorteilhaft dadurch ausgestaltet, daß auf dem Videoanzeigegerät verschiedene Abbildungen gleichzeitig zu Vergleichszwecken optisch sichtbar dargestellt werden. Ein Arbeiten nach diesem Verfahren dient besonders der Schonung der Zahnsubstanz, damit nicht durch Abtragen von Zahnsubstanz an

**0025911**

an falschen Stellen irreparable Fehlstellen entstehen. Auf einem Rechner kann z. B. der Idealzustand eines präparierten Zahnes oder Gebißteiles vorprogrammiert und gespeichert werden. Der Zahnarzt kann diesen Idealzustand ableiten aus einer Ist-Aufnahme, wenn er den gewünschten Abtrag von 1,8 bzw. 0,8 mm oder auch einen anderen notwendigen Betrag zum Abschleifen vorgibt. Wie auf einem Monitor können dann bei dem Präparieren zwischendurch immer Prüfungen vorgenommen werden, wobei der Ist-Zustand und der ideale Soll-Zustand deckungsgleich auf den Bildschirm projiziert werden, so daß der Zahnarzt sofort erkennt, an welchen Stellen Zahnsubstanz zu entfernen ist und an welchen nicht. Dadurch können Gebißregulierungen begünstigt werden, und es ist auch Zahnersatz über 20 Zähne gleichzeitig mit größter Genauigkeit ohne Gefahr möglich.

Die Vorrichtung zur Durchführung des Verfahrens ist durch eine optische Abbildungseinrichtung zum Bestreichen der betreffenden Zahnpartie, ferner einen elektronischen Rechner zum Abtasten und Auswerten der von der Abbildungseinrichtung erzeugten Abbildungsinformationssignale, durch eine Fräsmaschine und/oder gegebenenfalls durch ein Speichergerät gekennzeichnet. Die Anwendung dieser ansich bekannten Geräte in dieser Reihenfolge und zu dem genannten Zweck ist überraschend. Der Zahnarzt kann sich eine allgemeine bekannte Technik zunutze machen und mit wirtschaftlich vertretbaren Mitteln Erfolge erreichen, die an Schnelligkeit, Genauigkeit, angenehmer Behandlung des Patienten usw. bisher ihresgleichen suchen.

Dabei ist es von besonderem Vorteil, wenn erfindungsgemäß die optische Abbildungseinrichtung eine Halterung in Gestalt eines Abdrucklöffels für mehrere Linsen aufweist, an denen jeweils Lichtleitkabel befestigt sind, an deren anderem Ende eine Lichtquelle und eine Aufzeichnungseinrichtung angebracht sind, mit welcher eine elektronische Abtasteinrichtung verbunden ist, und wenn ein Rechner zur Auswertung und/oder Speicherung der Abbildungsinformationssignale angeschlossen ist. Es wurde eingangs die andere Alternative für die Abbildungseinrichtung, nämlich das Röntgenpanoramaaufnahmegerät beschrieben, welches so zu ergänzen ist, daß von mindestens drei Seiten die betreffende Zahnpartie betrachtet werden kann. Es sind lediglich entsprechende Motorantriebe vorzusehen, mit deren Hilfe die verschiedenen Röntgenaufnahmen, sich gegenseitig überlappend, von allen Seiten des Raumes aus auf den Zahnstumpf gerichtet, hergestellt werden.

Die andere Ausführungsform mit der rein optischen Photographie oder mit den Filmen läßt sich besonders zweckmäßig und leicht dadurch aufbauen, daß man Lichtleitkabel aus ebenfalls ansich bekanntem Fasermaterial einsetzt. Mit deren Hilfe ist es ohne weiteres möglich, die Linsen in einen solchen Bereich neben die zu photographierenden Zähne an ihren Platz zu bringen, daß mit einer Aufnahme z. B. das gesamte obere oder untere Gebiß rundum von allen Seiten photographiert wird. Dabei ist es zweckmäßig, wenn eine Auslöseeinrichtung mit der optischen Abbildungseinrichtung verbunden ist. Diese dient als Zeitgeber und löst verschiedene Aufnahmen in der Weise der bekannten Photogrammetrie aus. Auch hier sind die Bilder zweckmäßig wieder überlappend angeordnet.

Durch die erfindungsgemäßen Maßnahmen können die mehr oder weniger defekten natürlichen Zahnkronen in ihrer ursprünglichen Form wieder aufgebaut oder gelegentlich sogar zu einer funktionell noch günstigeren Form her-

-12-

gestellt werden. Mit der beschriebenen Technik kann nicht nur das Arbeitsmodell erstellt, sondern schnell und genau auch der temporäre Ersatz aufgebaut werden. Dieser dient zum Aufsetzen auf die Zahnstümpfe als Schutz gegen chemische und physikalische Einflüsse, z. B. gegen Säure und Zucker, Hitze und Kälte.

Die Hülsenkrone aus Metall findet Verwendung als Schutzkrone, als Ersatzkrone oder als Halteelement für Brückenarbeiten. Auch hier zeigt sich die günstige Wirkung der erfindungsgemäßen Maßnahmen: Das Präparieren des Zahnes erfolgt in bekannter Weise, indem mindestens 0,4 bis 0,5 mm als Kronendeckelstärke vom Zahn abgenommen wird. Alternativ verlangen Hülsenkronen aus Porzellan oder Kunststoff eine Stufenpräparation des Zahnes. In jedem Falle wird der Zahnstumpf oder werden die Zahnstümpfe präpariert und dann durch die erfindungsgemäßen Maßnahmen nachgebildet. Das Ersatzstück als Positiv wird auf dem Arbeitsmodell von Hand aus gut modellierfähigem Wachs beispielsweise modelliert. Ist dieses Ersatzstück fertiggestellt, dann kann es ebenso elektrooptisch erfaßt und gespeichert werden, und zwar gegebenenfalls mit großer Genauigkeit in dreidimensionaler Form, so daß eine Fräsmaschine den temporären Ersatz oder auch das Ersatzstück aus Wachs mit Hilfe von Steuersignalen fräsen kann, die der Fräsmaschine zuvor eingegeben sind. Deren Zustand richtet sich nach dem präparierten Gebißteil, dieser aber ist datenmäßig durch die oben beschriebenen Maßnahmen bereits in Gestalt kodierter Steuersignale vorhanden, z. B. auf einem Magnetband oder Lochstreifen gespeichert.

Weitere Vorteile, Merkmale und Anwendungsmöglichkeiten der vorliegenden Erfindung ergeben sich aus der folgenden Beschreibung im Zusammenhang mit den Zeichnungen. Es zeigen:

Fig. 1 ein Arbeitsmodell mit aufgesetzter Brücke,perspektivisch,

Fig. 2 ebenfalls perspektivisch und schematisiert eine
       Abbildungseinrichtung mit Lichtleitkabeln,

Fig. 3 schematisch die Anordnung der Gesamtvorrichtung,

Fig. 4 perspektivisch einen abgeschliffenen Zahnstumpf,

Fig. 5 perspektivisch ein modelliertes Ersatzstück aus
       Wachs, welches allerdings auf einen anderen
       Zahnstumpf, nicht auf den nach Figur 4, paßt,und

Fig. 6 in perspektivischer Darstellung das Aufsetzen
       einer längeren Brücke auf ein Arbeitsmodell in
       spezieller Parallelführung.

Zur Veranschaulichung der Vorrichtung zum Herstellen
von Zahnersatz wird im folgenden eine bevorzugte Ausführungsform beschrieben, nämlich mit Hilfe der photographischen Methode, nach der Photogrammetrie.

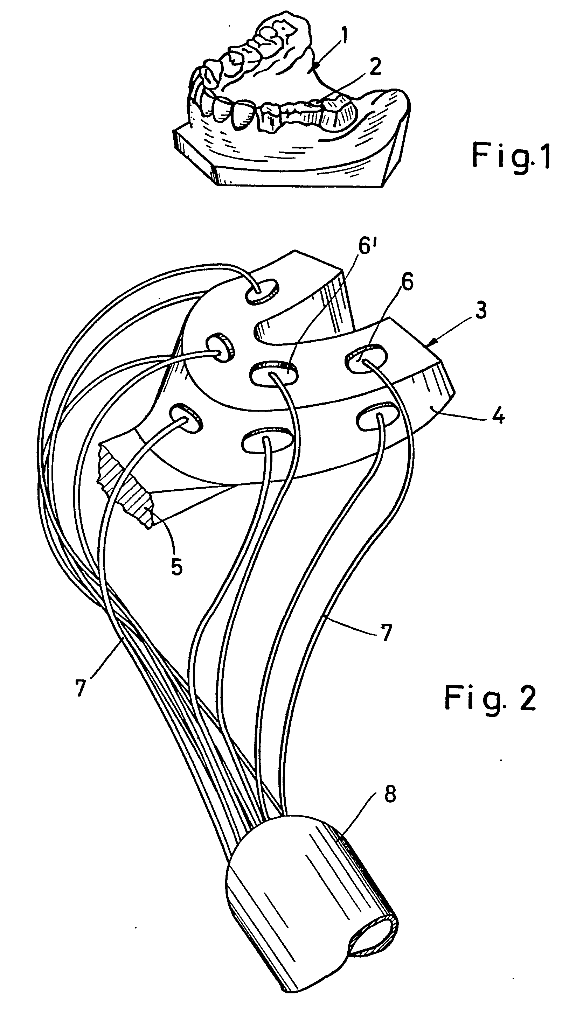

In Figur 1 ist perspektivisch ein Arbeitsmodell 1
eines Unterkiefers gezeigt, bei welchem vorn rechts
eine Brücke 2 aufgesetzt ist.

In Figur 2 ist perspektivisch die allgemein mit 3 bezeichnete optische Abbildungseinrichtung dargestellt,
die eine Halterung 4 in Gestalt eines Abdrucklöffels
aufweist, der gerade über den Unterkiefer gemäß Figur
1 paßt. Mit einem abgebrochen gezeigten Löffelstiel 5
wird diese Halterung 4 über die Zahnreihe mit den
Nachbarbereichen gestülpt. In gewissen Abständen ist auf allen Oberflächen eine große Anzahl von schematisch dargestellten Linsen 6 an der Halterung 4 befestigt,von denen in der Darstellung der Figur 2 lediglich vier Linsen 6 auf der hufeisenförmigen Oberfläche und drei Linsen auf der U-förmigen, daran anschließenden Fläche dargestellt sind. Es versteht sich aber, daß auch von der Innenseite, d. h. vom Gaumen her, sowie auch auf der Rückseite entsprechende Halterungen für Linsen 6 vorgesehen sind. Die Linsen sind optisch so vorbereitet,daß sie sich gegenseitig überlappende Bilder von dem gerade in der Nachbarschaft befindlichen Kieferbereich mit Zähnen abbilden, so daß also die Linse 6 ein Bild erzeugt,welches sich mit dem der Linse 6' mindestens teilweise überschneidet. Diese Regel gilt auch für alle anderen Linsen.

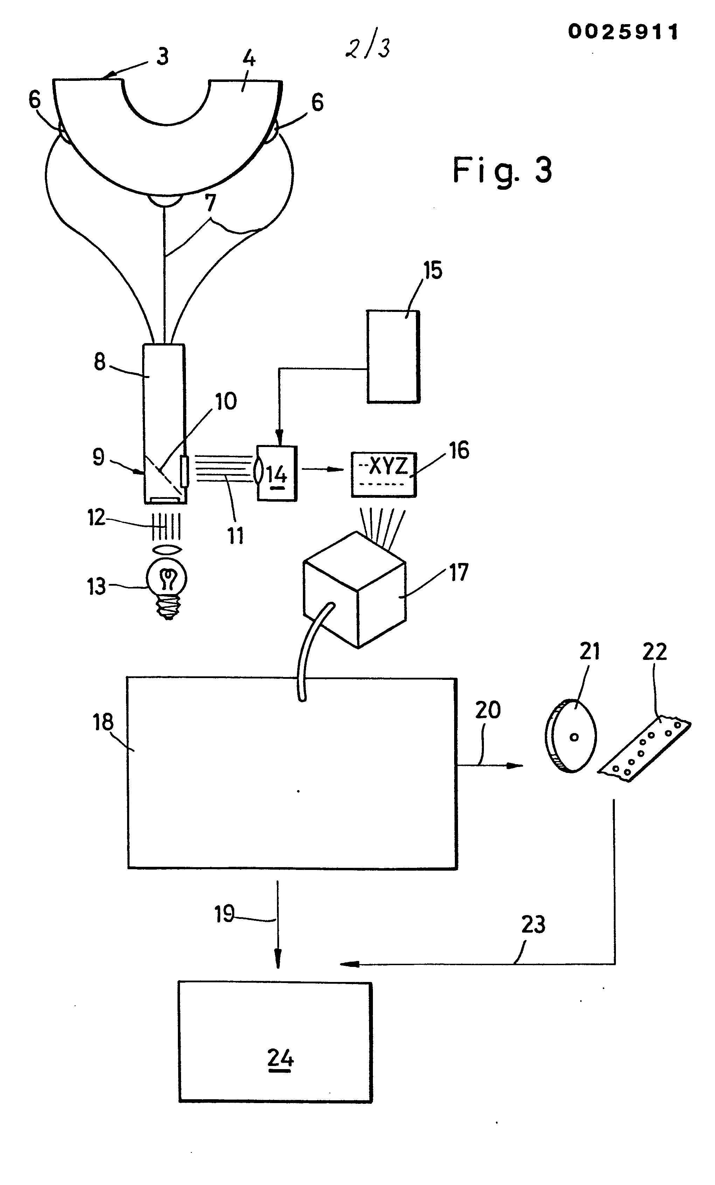

Lichtleitkabel 7 führen von den Linsen 6, 6' durch ein Sammelkabel 8 zu einer schematisch in Figur 3 dargestellten, allgemein mit 9 bezeichneten Einrichtung, in der eine halb verspiegelte Fläche 10 für den Durchtritt eines Teils von Strahlen 11 und eines Teils von Strahlen 12 erlaubt. Die Lichtquelle 13 erzeugt Lichtstrahlen 12, welche durch die halb verspiegelte Fläche 10 hindurchtreten und mit Hilfe der Linsen 6, 6' die Beleuchtung der betreffenden Zahnpartie sicherstellen. Das Licht kommt von den belichteten Zahnpartien durch die Lichtleitkabel 7 aus der Vorrichtung 9 in den Photoapparat 14, dessen Blende durch eine Auslöseeinrichtung 15 getaktet wird. Dadurch entstehen photographische Abbildungen, die in Fig. 3 bei 16 schematisch dargestellt sind. Sie werden von der Abtasteinrichtung 17 abgetastet zur Erzeugung von Abbildungsinformationssignalen, die danach in den elektronischen Rechner 18 eingegeben werden. In diesem werden die damit dem betreffenden Zahnstumpf entsprechenden Abbildungsinformationssignale umgeformt und gelangen als elektrische und/oder magnetische Steuersignale entweder direkt über den Weg 19 oder indirekt nach Einspeichern über den Weg 20 in das Magnetband 21 oder den Lochstreifen 22 und dann über den Weg 23 zu einer automatischen Fräsmaschine 24.

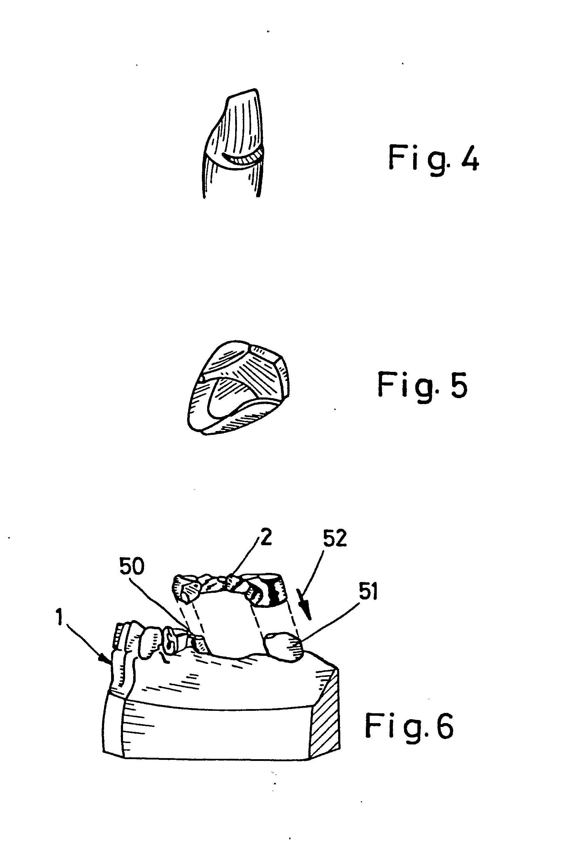

Lediglich zur Veranschaulichung ist in Figur 4 perspektivisch ein präparierter Zahnstumpf gezeigt,der eine labial eingeschliffene, zervikale Schulter aufweist.

In Figur 5 ist für eine andere Form von Zahnstumpf ein modelliertes Wachspositiv gezeigt. Um dieses wird in bekannter Weise ein Gipsbehälter geformt, das Wachs wird ausgebrannt, und dann wird Metall, vorzugsweise Gold, zur Herstellung eines ebenso gestalteten Formlings, wie in Figur 5 gezeigt,aus Metall eingefüllt.

Das Fräsen aus Wachs oder eines temporären Ersatzes kann erfindungsgemäß ohne weitere mit Hilfe der elektronisch angesteuerten Fräsmaschine erfolgen, welche die Steuersignale von einem Rechner oder aus einem Speicher erhält, in den sie in der oben beschriebenen Weise eingegeben wurden, nämlich nach der Röntgenaufnahme oder der Photogrammetrie.

In Figur 6 ist schließlich perspektivisch ein Arbeitsmodell 1 gezeigt, bei welchem die Brücke 2 erst aufgesetzt werden soll. Man erkennt zwei Zahnstümpfe 50 und 51, die zuvor entsprechend den gestrichelten Linien und entsprechend dem Pfeil 52 genau parallel zu präparieren waren. Diese Parallelgestaltung der Pfeiler ist nämlich erforderlich, um die Eingliederung dieser mehrgliedrigen Brücke zu ermöglichen. Je grösser die Zahl der Pfeiler ist, umso schwieriger wird es, eine Parallelität aller Anker zu erzielen. Erfindungsgemäß wird diese Parallelität aber durch die oben beschriebenen Maßnahmen schnell und sehr genau dadurch erreicht, daß die Abbildungsinformationssignale für eine optisch sichtbare Abbildung auf einem Videoanzeigegerät ausgewertet werden. Bei entsprechender Vergrößerung kann hier die Parallelität während des Präparierens jederzeit und sehr genau überprüft werden. Um Zahnsubstanz zu schonen und die Pulpe nicht zu verletzen, kann durch die gleichzeitige Darstellung einer vorher eingegebenen Soll-Aufnahme mit der jeweiligen Ist-Aufnahme auf dem Videoanzeigegerät zu Vergleichszwecken exakt die Materialdicke vom Zahn abgetragen werden, die zum Aufsetzen der Brücke 2 erforderlich ist.

Durch die erfindungsgemäßen Maßnahmen können die Arbeiten im Behandlungszimmer schnell und genau durchgeführt werden. Man braucht dort lediglich die Abbildungseinrichtung 3, die Lichtleitkabel 7, die Vorrichtung 9, die Lichtquelle 13 und den Photoapparat 14, gegebenenfalls die Auslöseeinrichtung 15.

Die in Figur 3 mit 16 bezeichnete Photographie bzw. der Film oder die Röntgenplatte kann dann im Labor ausgewertet, kodiert und in eine Fräsmaschine gegeben werden, die dann das gewünschte Arbeitsmodell oder den Zahnersatz aus dem vorgegebenen Materialblock herausfräst.

Durch die Erfindung ist auch die Speicherung bzw. Archivierung in sehr platzsparender Weise möglich, so daß sämtliche Daten, die ein Zahnarzt von seinen Patienten benötigt, jederzeit abrufbar zur Verfügung stehen. Damit ist es z. B. möglich, die räumlichen Koordinaten des jungen, unbeschädigten Gebisses eines Patienten mittels Photogrammetrie zu archivieren und danach später gegebenenfalls Zahnersatz nachzubilden. Auch für die Kriminologie ist ein solches umfangreiches Archiv von großer Bedeutung.

**Patentansprüche**

1. Verfahren zur Herstellung von Zahnersatz, bei dem der präparierte Zahnstumpf in einem Arbeitsmodell nachgebildet und das von Hand an das Arbeitsmodell entsprechend angepaßte Ersatzstück geformt wird, d a d u r c h   g e k e n n z e i c h n e t , daß

a) die Nachbildung des Zahnstumpfes elektrooptisch derart erfolgt, daß dem Zahnstumpf entsprechende Abbildungsinformationssignale erzeugt werden, die in einem elektronischen Rechner zu elektrischen und/oder magnetischen Steuersignalen umgeformt werden, mit deren Hilfe eine Fräsmaschine das Arbeitsmodell aus einem Materialblock selbsttätig herausfräst, und/oder

b) daß das positive Ersatzstück von der Fräsmaschine nach Steuersignalen aus einem Materialblock herausgefräst wird, die aus Abbildungsinformationssignalen abgeleitet sind, welche elektrooptisch von dem handgeformten Ersatzmodell abgeleitet sind.

2. Verfahren nach Anspruch 1, dadurch gekennzeichnet, daß die elektrooptische Nachbildung des Zahnstumpfes und/oder Ersatzmodells photogrammetrisch oder durch Röntgenaufnahmen erfolgt und daß die Abbildungsinformationssignale von einem Papierphoto, einem optischen Film, einer Röntgenplatte oder einem Videoband abgeleitet werden.

3. Verfahren nach Anspruch 1 oder 2, dadurch gekennzeichnet, daß die elektrischen und/oder magnetischen Steuersignale zwischengespeichert werden.

4. Verfahren nach einem der Ansprüche 1 bis 3, dadurch gekennzeichnet, daß die Abbildungsinformationssignale für eine optisch sichtbare Abbildung auf einem Videoanzeigegerät ausgewertet werden.

5. Verfahren nach einem der Ansprüche 1 bis 4, dadurch gekennzeichnet, daß auf dem Videoanzeigegerät verschiedene Abbildungen gleichzeitig zu Vergleichszwecken optisch sichtbar dargestellt werden.

6. Vorrichtung zur Durchführung des Verfahrens nach einem der Ansprüche 1 bis 5, dadurch gekennzeichnet, daß eine optische Abbildungsaufnahmeeinrichtung (3) zum Bestreichen der betreffenden Zahnpartie vorgesehen ist, ferner ein elektronischer Rechner (17, 18) zum Abtasten und Auswerten der von einer Abbildungseinrichtung(14) erzeugten Abbildungsinformationssignale, eine Fräsmaschine (24) und/oder gegebenenfalls ein Speichergerät (21, 22) vorgesehen sind.

6. Vorrichtung nach Anspruch 6, dadurch gekennzeichnet, daß die optische Abbildungsaufnahmeeinrichtung (3) eine Halterung (4) in Gestalt eines Abdrucklöffels für mehrere Linsen (6, 6') aufweist, an denen jeweils Lichtleitkabel (7) befestigt sind, an deren anderem Ende eine Lichtquelle (13) und eine Aufzeichnungseinrichtung (14) angebracht sind, mit welcher eine elektronische Abtasteinrichtung (17) verbunden ist, und daß ein Rechner (18) zum Auswerten und/oder Speichern der Abbildungsinformationssignale angeschlossen ist.

8. Vorrichtung nach Anspruch 6 oder 7, dadurch gekennzeichnet, daß eine Auslöseeinrichtung (15) mit der optischen Abbildungsaufzeichnungseinrichtung(14)ver-

bunden ist.

Fig.1

Fig. 2

0025911

Fig. 3

Fig. 4

Fig. 5

Fig. 6

**Europäisches Patentamt**

# EUROPÄISCHER RECHERCHENBERICHT

| | EINSCHLÄGIGE DOKUMENTE | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| X | US - A - 3 861 044 (SWINSON, JR.)<br>* ganzes Dokument *<br>-- | 1,2,6 | A 61 C 9/00<br>A 61 C 19/04<br>A 61 B 5/10 |
| A | US - A - 3 100 344 (SHARP)<br>---- | | |

RECHERCHIERTE SACHGEBIETE (Int. Cl.3)

A 61 B 5/00
A 61 C 9/00
A 61 C 13/00
A 61 C 19/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 18-12-1980 | SIMON |

EPA form 1503.1   06.78